# EUROPÄISCHE PATENTSCHRIFT

(11) **EP 1 193 239 B1**
(45) Veröffentlichungstag und Bekanntmachung des Hinweises auf die Patenterteilung: **08.12.2004**
(21) Anmeldenummer: 01119282.0
(22) Anmeldetag: 10.08.2001
(51) Int. Cl.: C07C 45/50, B01J 31/40

(54) **Stabilisierung von Rhodiumkatalysatoren für die Hydroformylierung von Olefinen**
Stabilisation of rhodium catalysts for the hydroformylation of olefins
Stabilisation de catalyseurs à base de rhodium pour l'hydroformylation d'olefines

(30) Priorität: 29.09.2000 DE 10048301
(43) Veröffentlichungstag der Anmeldung: 03.04.2002
(73) Patentinhaber: Oxeno Olefinchemie GmbH, 45772 Marl (DE)
(72) Erfinder: Wiese, Klaus-Diether, Dr., 45721 Haltern (DE); Trocha, Martin, Dr., 45136 Essen (DE); Röttger, Dirk, Dr., 45657 Recklinghausen (DE); Tötsch, Walter, Dr., 45770 Marl (DE); Kaizik, Alfred, Dr., 45772 Marl (DE); Büschken, Wilfried, Dr., 45721 Haltern (DE)

(56) Entgegenhaltungen:
- EP-A- 0 649 851
- WO-A-97/30016
- US-A- 4 041 082
- US-A- 4 178 313

## Beschreibung

Die vorliegende Erfindung betrifft ein verbessertes Verfahren zur Herstellung von Aldehyden durch Hydroformylierung von Olefinen durch Verringerung der Katalysatordesaktivierung bei der Aufarbeitung von Rhodium-Katalysatoren.

In technischem Maßstab werden Hydroformylierungen von Olefinen mit Kobalt- oder mit Rhodiumkatalysatoren durchgeführt Dabei ist die Verwendung von Rhodiumkatalysatoren meist vorteilhaft, da mit ihnen höhere Selektivitäten und Produktausbeuten erreicht werden können. Im Vergleich zum Kobalt ist Rhodium jedoch teurer; bei der Hydroformylierung von Olefinen zu den entsprechenden Aldehyden mit Rhodium-Katalysatoren ist der Katalysator ein nicht unbedeutender Kostenfaktor. Zur Erhöhung der Wirtschaftlichkeit muß der spezifische Katalysatorverbrauch gesenkt werden. Darunter ist die Menge an Katalysator zu verstehen, die bei einem Langzeitbetrieb dem Prozeß zugeführt werden muß, um ein konstantes Aktivitätsniveau zu gewährleisten.

Die Rhodium-katalysierte Umsetzung von Olefinen zu den entsprechenden Aldehyden erfolgt meist in homogener flüssiger Phase. Bei der Hydroformylierung von Propen hat sich inzwischen auch ein Verfahren etabliert, bei dem der Katalysator in einer zweiten flüssigen Phase gelöst vorliegt; die Anwendbarkeit dieses Verfahrens auf längerkettige Olefine ist aber begrenzt.

Bei Hydroformylierungen in homogener Phase, d. h. Katalysator, Olefine, Produkte, Lösungsmittel usw. liegen in einer Phase vor, stellt sich das Problem, den Katalysator nach der Reaktion von den Produkten abzutrennen. Dies kann einfach über eine Abdestillation des nicht umgesetzten Edukts und der Produkte erfolgen; der im Sumpf, meist in hochsiedenden Komponenten gelöste Katalysator, wird anschließend in den Reaktor zurückgeführt. Die Destillation kann dabei kontinuierlich oder diskontinuierlich erfolgen.

Bei der destillativen Abtrennung ist häufig eine Zersetzung oder Desaktivierung des Katalysators festzustellen. Besonders bei der Hydroformylierung längerkettiger Olefine kann die Destillation aufgrund der Siedepunkte der Produkte nur noch bei erhöhter Temperatur und/oder vermindertem Druck durchgeführt werden.

Für die Verminderung der Rhodiumdesaktivierung während der Aufarbeitung des Reaktoraustrags bei Hydroformylierungen sind mehrere Methoden bekannt.

In EP 0272608 B1 ist ein Verfahren beschrieben, in dem für die Hydroformylierung ein Rhodiumkatalysator mit Triphenylphosphinoxid-Liganden eingesetzt wird. Bei der Aufarbeitung des Reaktionsaustrags wird vor dessen Destillation Triphenylphosphin (die neunfache Menge bezogen auf Rhodium) zugesetzt. Der Destillationsrückstand enthält Rhodiumkomplexe mit Triphenylphoshin als Liganden sowie Triphenylphoshin und Triphenylphosphinoxid. In diesem Gemisch wird das freie und komplexierte Triphenylphosphin zu Triphenylphosphinoxid oxidiert. Diese Katalysatorlösung wird in den Reaktor zurückgeführt. Für die Oxidation des Triphenylphosphins wird Sauerstoff oder ein Peroxid eingesetzt. Weitere Varianten dieser Methode sind bekannt und in JP 63 222 139, JP 63 208 540, DE 3 338 340 und JP 63 218 640 beschrieben.

Diese Verfahren haben folgende Nachteile: Es wird ständig Triphenylphosphin verbraucht. Daraus entsteht durch Oxidation die äquivalente Menge Triphenylphosphinoxid. Um dessen Konzentration im Reaktor zu begrenzen, ist ein Ausschleusestrom notwendig, durch den wiederum Rhodium ausgetragen wird. Zusätzlich ist eine Oxidationsvorrichtung notwendig. Bei der Oxidation fallen, wenn nicht gerade Luft verwendet wird, Kosten für das Oxidationsmittel an.

In der einschlägigen Literatur (z. B. US 4 400 547) sind weitere Verfahren, die andere Phosphorliganden zur Stabilisierung des Rhodiums einsetzen, beschrieben.

In den Patentschriften US 5 731 472, US 5 767 321 und EP 0 149 894 werden Verfahren zur Hydroformylierung von n-Butenen beschrieben. Darin werden Rhodium-Katalysatoren eingesetzt, die Phosphit-Liganden enthalten und durch Zusatz von Aminen stabilisiert sind. Nachteilig daran ist, dass Amine als Katalysatoren für Aldolkondensationen wirken können und so die Bildung von Hochsiedern begünstigt wird.

Die Hydroformylierung von einem C₈-Olefingemisch, hergestellt durch Dimerisierung von Butenen, unter der Katalyse von Rhodiumkomplexen und deren Stabilisierung mit substituierten Phenolen wird in JP-04-164042 beschrieben. Dabei werden Rhodiumverbindung, Ligand und Stabilisator im molaren Verhältnis 1/10/50 eingesetzt. Nachteilig bei diesem Verfahren sind die Kosten für den Stabilisator und der Aufwand für dessen Abtrennung.

Es bestand daher die Aufgabe, ein Verfahren zur Hydroformylierung von Olefinen zu entwickeln, bei dem die Desaktivierung des Rhodiumkatalysators weitgehend unterdrückt wird.

Gegenstand der vorliegenden Erfindung ist daher ein Verfahren zur Herstellung von Aldehyden mit 3 bis 21 Kohlenstoffatomen durch Hydroformylierung der entsprechenden Olefine mit Rhodiumkatalysatoren, wobei der Austrag des Hydroformylierungsreaktors
a) in eine Gasphase und eine Flüssigphase getrennt,
b) die Flüssigphase in eine Kopffraktion, enthaltend nicht umgesetzte Olefine und Aldehyde, und eine Sumpffraktion enthaltend den Rhodiumkatalysator getrennt und
c) die Sumpffraktion unter die Temperatur des Austrags des Hydroformylierungsreaktors auf eine Temperatur von 10 - 120°C abgekühlt und mit einem Kohlenmonoxid-haltigen Gas mit einem Kohlenmonoxidpartialdruck von 5 - 300 bar beaufschlagt
wird.

Durch das erfindungsgemäße Verfahren kann der Aktivitätsverlust des Katalysators während der Aufarbeitung des Hydroformylierungsaustrags drastisch reduziert werden. Überraschenderweise wurde gefunden, dass mit Kohlenmonoxid stabilisierte Rhodium-Katalysatorlösung über Wochen lagerstabil sind.

Die erfindungsgemäßen Verfahren haben gegenüber bekannten Verfahren folgende Vorteile: Der Katalysator wird während der Aufarbeitung kaum desaktiviert. Es werden keine zusätzlichen Stoffe benötigt, die das Verfahren durch ihre Stoffkosten belasten. Der Katalysator wird mit einem Stoff stabilisiert, der sowieso im Reaktor vorhanden ist. Es ist möglich, die Katalysatorlösung ohne Aktivitätsverlust zu lagern. Dies ist insbesondere bei längerfristigen Abstellungen, wie z. B. bei Großreparaturen oder Revisionen, oder bei der kampagnenweise Herstellung von Produkten, von Vorteil.

Die Hydroformylierung wird in homogener Phase in einem Reaktor nach bekannten Verfahren (B. Cornils, W. A. Herrmann, "Applied Homogeneous Catalysis with Organometallic Compounds", Vol. 1&2, VCH, Weinheim, New York, 1996) durchgeführt. Als Edukte kommen alle Olefine mit 2 bis 20 Kohlenstoffatomen in Frage, insbesondere Butene, Penten, Hexene und Octene, hier besonders das durch Butenoligomerisierung erhaltene Dibuten. Der Produktstrom, bestehend aus Aldehyden, Alkoholen, nicht umgesetzten Olefinen, Hochsiedern, Katalysatorsystem, Neben- und Zersetzungsprodukten wird zuerst in einer Trennstufe, Verfahrensstufe a), in eine Gas- und eine Flüssigphase aufgetrennt. Die Gasphase enthält dabei den größten Teil des nicht umgesetzten Synthesegases und je nach Temperatur und Druck Anteile an nicht umgesetzten Olefinen, Aldehyden, Kohlenwasserstoffen und anderen Bestandteilen. Die Flüssigphase besteht hingegen hauptsächlich aus den Hydroformylierungsprodukten und nicht umgesetzten Olefinen. Die Temperatur in dieser Trennstufe beträgt 30-180 °C, bevorzugt 50-150 °C. Die Trennung findet unter einem Partialdruck von Kohlenmonoxid von 0,5 bis 100 bar statt, bevorzugt bei 1 bis 35 bar. Dadurch wird auch in diesem Anlagenteil eine Stabilisierung des Rhodiums gewährleistet. Technisch kann diese Trennung sowohl am Kopf des Hydroformylierungsreaktors, als auch in einem separaten Apparat, z. B. in einem Flasher, erfolgen. Wird der Reaktor bei höherem Druck als die Trennstufe betrieben, erfolgt zwischen diesen eine Entspannung. Der Kohlenmonoxidpartialdruck kann entweder durch das im Hydroformylierungsreaktor eingesetzte Gasgemisch oder durch Zugabe eines Kohlenmonoxid-haltigen Gases aufrechterhalten werden.

Da der Katalysator mit noch nicht umgesetzten Olefinen weiter reagieren kann, ist wegen einer möglichen Verarmung der Flüssigphase an Synthesegas die Gefahr der Katalysatorzersetzung erhöht und somit in dieser Trennstufe eine kurze Verweilzeit der flüssigen Phase anzustreben. Günstig sind Verweilzeiten unter 30 bevorzugt unter 15 Minuten.

Nach der Trennung in Gas und Flüssigkeit wird die flüssige Phase destillativ in eine Kopfund eine Sumpffraktion aufgetrennt (Fraktionierungsstufe, Verfahrensstufe b)). In der Sumpffraktion befindet sich dabei der Katalysator, gelöst in Hochsiedern, die entweder dem Prozess zugesetzt und/oder im Prozess gebildet werden. In der niedriger siedenden Kopffraktion befinden sich hauptsächlich die Oxo-Produkte und die nicht umgesetzten Olefine.

Die mittlere Verweilzeit der Flüssigphase in der Fraktionierungsstufe liegt unter 15 Minuten, bevorzugt unter 5 Minuten, besonders bevorzugt unter 2 Minuten. Die Fraktionierungsstufe b) kann zur Trennung einen Flasher, einen Fallfilmverdampfer, einen Dünnschichtverdampfer oder vergleichbare Apparate aufweisen, die eine schonende Abtrennung ermöglichen. Auch Kombinationen dieser Einheiten können eingesetzt werden, so zum Beispiel ein Fallfilmverdampfer, dessen Sumpfablauf in einen Dünnschichtverdampfer überführt wird.

Der Druck in der Fraktionierungsstufe liegt zwischen 0.01 mbar und 1 bar, vorzugsweise zwischen 10 mbar und 1 bar. Die Temperatur beträgt 40 °C bis 180 °C, bevorzugt 80 °C bis 150 °C. Die aus der Fraktionierungsstufe austretende Sumpffraktion wird umgehend auf Temperaturen von 10 °C bis 120 °C heruntergekühlt, bevorzugt auf Temperaturen von 40 °C bis 90 °C und unter einen Kohlenmonoxidpartialdruck von 5 bar bis 300 bar, insbesondere 5 bis 64 bar gestellt. Als Kohlenmonoxid-haltiges Gas kann reines Kohlenmonoxid, Synthesegas oder sonstige Gemische von Kohlenmonoxid mit inerten Gasen, z. B. Stickstoff, Kohlendioxid, Wasserstoff und/oder Methan, genutzt werden.

Eine mögliche Ausführung dieses Verfahrensteils ist es, den Hochsieder aus der Fraktionierungsstufe in einem Kühler oder alternativ durch Mischen mit einer kühleren Flüssigkeit, vorzugsweise mit dem Einsatzolefin, abzukühlen und ihn dann mittels einer Pumpe in einen mit Kohlenmonoxid beaufschlagten Behälter, wie zum Beispiel einen Rührkessel, ein Druckgefäß oder eine Druckleitung, zu pumpen.

Die Lagerung der Katalysatorlösungen geschieht bevorzugt bei Temperaturen, die geringer als die Ablauftemperaturen der Katalysatorlösung aus der Verfahrensstufe b) sind. Bevorzugte Lagertemperaturen der Sumpffraktion sind daher 10 bis 120 °C, insbesondere 40 bis 90 °C. Optional kann der zu lagernden Katalysatorlösung ein Solvens zugesetzt werden, zweckmäßig ein Stoff, der im Prozess vorhanden ist, wie beispielsweise Edukt (Olefin), Produkt (Aldehyd) oder hydriertes Produkt (Alkohol).

Diese Katalysatorlösung, d. h. die Sumpffraktion der Verfahrensstufe b) kann ganz oder teilweise in den Hydroformylierungreaktor zurückgeführt werden. Die in der Fraktionierungsstufe b) anfallenden Brüden, d. h. nicht umgesetztes Olefin und die Produkte der Hydroformylierung werden nach bekannten Verfahren aufgearbeitet.

Die folgenden Beispiele sollen die Erfindung beschreiben, ohne deren Schutzbereich, wie in den Patentansprüchen definiert, einzuschränken.

### Beispiel 1

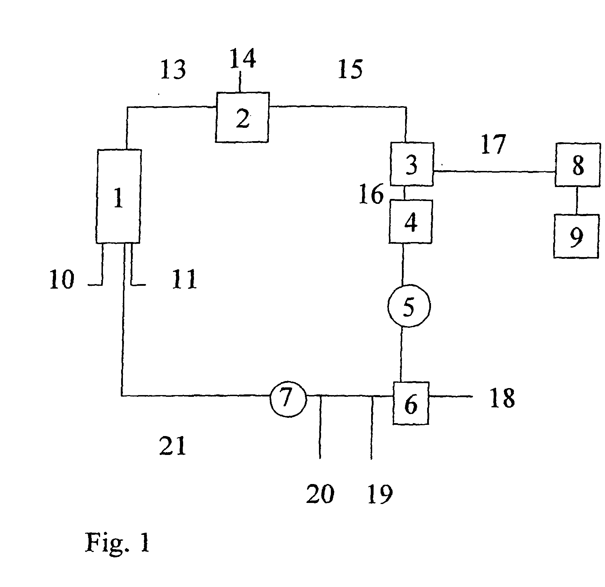

In einer technischen Versuchsanlage (Fig. 1) wurde wie folgt hydroformyliert:

In den Blasensäulenreaktor (1) (Volumen 60 l) wurden Olefin (10), Synthesegas (11) und Katalysatorlösung (21) eingespeist. Der Hydroformylierungsaustrag (13) wurde in einem Flash (2) auf 5 bar entspannt. Das entweichende Gas (14) wurde in einem nicht dargestellten Kühler abgekühlt und das dabei anfallende Kondensat mit der Flüssigkeit (15) vereinigt. Die im Flashbehälter (2) anfallende Flüssigphase (15) wird im Dünnschichtverdampfer (3) in eine Kopffraktion (17) und eine Sumpffraktion (16) getrennt. Das Rohprodukt (17) wird im Kühler (8) kondensiert und im Behälter 9 gesammelt. Das Sumpfprodukt (16), das den Katalysator, in Hochsiedern gelöst, enthält, wird im Kühler (4) abgekühlt (siehe Tabelle 3) und mit Hilfe der Pumpe (5) in den Zwischenbehälter (6) gefördert. Im Behälter (6) wird mit Synthesegas (18) ein Druck von 10 bar eingestellt. Die Temperatur der Katalysatorlösung (16) im Behälter (6) wurde gemäß Tabelle 3 bestimmt. Die Katalysatorlösung (16) konnte durch Ausschleusen einer Teilmenge (19) und Zugabe von Katalysatorvorläufer (Rhodiumverbindung und Ligand) (20) auf die gewünschte Aktivität im Reaktor (1) gebracht werden und wurde als Lösung (21) mit Pumpe (7) in den Hydroformylierungsreaktor (1) zurückgeführt.

| | | |
|---|---|---|
| Olefin | Di-n-buten | 5 kg/h |
| Synthesegas | CO/H₂(1/1) | 2 kg/h |
| Rhodiumverbindung | Rhodiumoctanoat | (30-90 ppm Rh im Reaktor 1) |
| Ligand | Tris(24-di-tert.-butylphenyl)phosphit | ( 20 mol Ligand/ mol Rh) |

In Tabelle 2 sind die Versuchsparameter, die während des gesamten Versuchs konstant gehalten wurden, dargestellt.

| | |
|---|---|
| Druck im Reaktor 1 | 50 bar |
| Temperatur im Reaktor 1 | 130 °C |
| Druck im Dünnschichtverdampfer 3 | 60 mbar |
| Temperatur im Dünnschichtverdampfer 3 (Austrittstemperatur am Sumpf) | 140 °C |

Die Aktivität des Katalysators wurde über den im Reaktor erreichten Umsatz überwacht. Sobald der Umsatz an Olefin 95 % unterschritt, wurde ein Teil der Katalysatorlösung aus Behälter (6) ausgeschleust und durch frischen Katalysatorvorläufer (Rhodiumsalz und Ligand) ersetzt, so dass der Umsatz wieder über 95 % lag. Ebenfalls wurde ein geringer Katalysatorverlust durch den Hochsiederaustrag wieder ersetzt.

Bei unterschiedlichen Temperaturen des Kühlers (4) (Austrittstemperatur Katalysatorlösung) mussten folgende Rhodiummengen (gerechnet als Metall) nachdosiert werden, um das Umsatzniveau zu halten (Tabelle 3):

| Temperatur Kühler 4 | Temperatur Behälter 6 | Rhodium (g), je Tonne umgesetztes Olefin |
|---|---|---|
| Keine Kühlung | 70 - 90 °C | 2,1 |
| 60 °C | 40 - 55 °C | 0,9 |

### Beispiel 2

### Rückgang der Katalysatoraktivität in Abhängigkeit vom Synthesegasdruck

In einem 3 l Autoklav (Büchi) wurden 350 g Toluol, 3.03 g Tris(2.4-di-tertbutylphenyl)phosphit und 0.096 g Rhodiumoctanoat unter 50 bar Synthesegasdruck (1/1 CO/H₂) eine Stunde bei 120 °C präformiert. Dann wurde eine Probe entnommen und die Aktivität des Katalysators in einem zweiten Autoklaven durch eine Hydroformylierungsreaktion mit Cycloocten (bei 120 °C, 50 bar Synthesegasdruck) bestimmt. Anschließend wurde der Katalysator im ersten Autoklav über einen Zeitraum von mehreren Stunden thermisch belastet; während dieser Zeit wurden Proben entnommen und wie bei der Bestimmung der Anfangsaktivität auf die Aktivität des Katalysators getestet. Der Versuch wurde bei verschiedenen Temperaturen und Synthesegasdrücken wiederholt.

Im Diagramm 1 ist der Einfluss des Synthesegasdrucks auf die Aktivität des Katalysators wiedergegeben (normierte Aktivität, frischer Katalysator hat die Aktivität 100 % bzw. 1). Bei einem Synthesegasdruck von 50 bar ist auch nach über 100 Stunden noch mehr als 80 % der Anfangsaktivität vorhanden, bei 20 bar Synthesegasdruck fällt die Aktivität schon nach 65 Stunden unter 40 % der Anfangsaktivität.

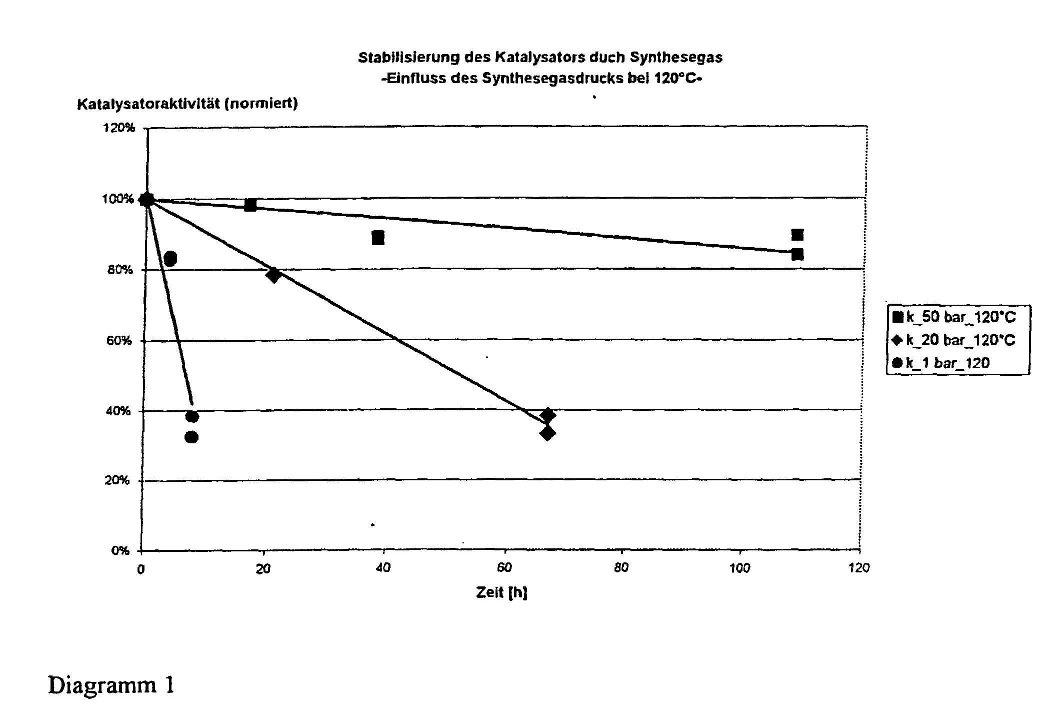

Im Diagramm 2 ist der Einfluss der Temperatur auf die Katalysatorstabilität bei einem konstanten Synthesegasdruck von 50 bar dargestellt. Eine Temperaturerhöhung von 120 °C auf 140 °C führt zu einer starken Beschleunigung der Zersetzung des Katalysators.

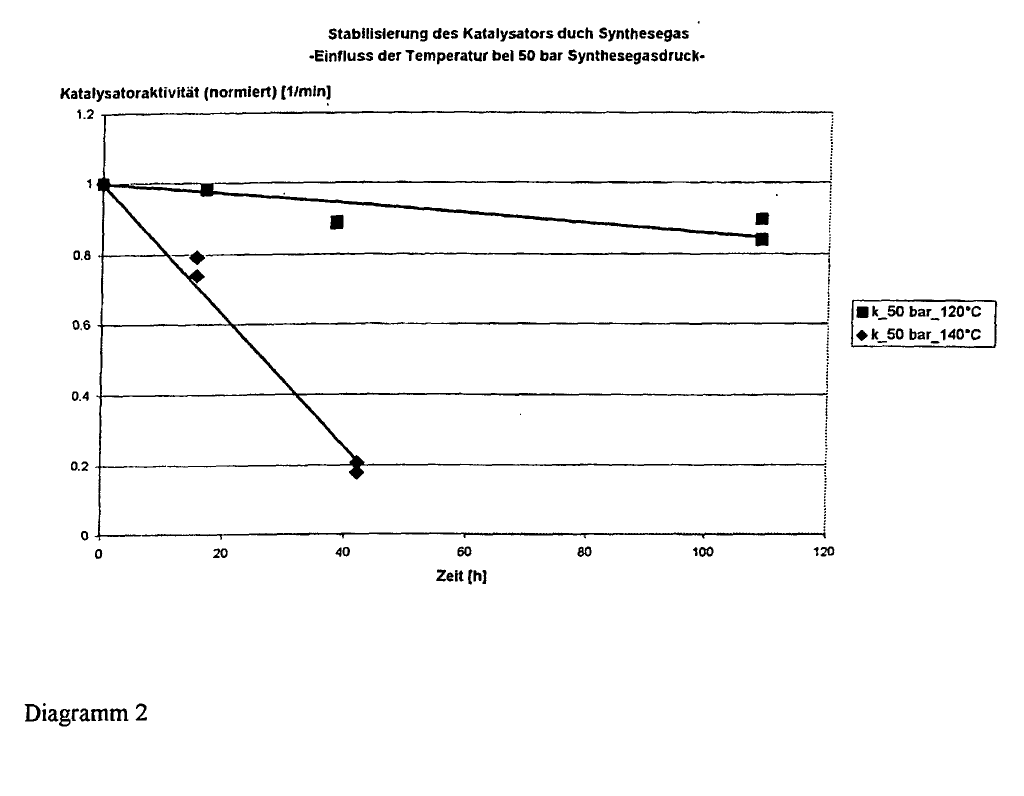

## Patentansprüche

1. Verfahren zur Herstellung von Aldehyden mit 3 bis 21 Kohlenstoffatomen durch Hydroformylierung der entsprechenden Olefine mit Rhodiumkatalysatoren,
**dadurch gekennzeichnet,**
**dass** der Austrag des Hydroformylierungsreaktors
a) in eine Gasphase und eine Flüssigphase getrennt,
b) die Flüssigphase in eine Kopffraktion, enthaltend nicht umgesetzte Olefine und Aldehyde, und eine Sumpffraktion enthaltend den Rhodiumkatalysator getrennt und
c) die Sumpffraktion unter die Temperatur des Austrags des Hydroformylierungsreaktors auf eine Temperatur von 10 bis 120 °C abgekühlt und mit einem Kohlenmonoxid-haltigen Gas mit einem Kohlenmonoxidpartialdruck von 5 bis 300 bar beaufschlagt
wird.

2. Verfahren nach Anspruch 1,
**dadurch gekennzeichnet,**
**dass** in der Verfahrensstufe a) ein Kohlenmonoxidpartialdruck von 0,5 bis 100 bar eingestellt wird.

3. Verfahren nach einem der Ansprüche 1 oder 2,
**dadurch gekennzeichnet,**
**dass** die Verfahrensstufe b) einen Fallfilmverdampfer, einen Dünnschichtverdampfer, einen Flasher oder eine Kombination dieser Einheiten aufweist.

4. Verfahren nach einem der Ansprüche 1 bis 3,
**dadurch gekennzeichnet,**
**dass** die mittlere Verweilzeit der Flüssigphase in der Verfahrensstufe b) unter 15 Minuten beträgt.

5. Verfahren nach einem der Ansprüche 1 bis 4,
**dadurch gekennzeichnet,**
**dass** die mittlere Verweilzeit der Sumpffraktion der Verfahrensstufe b) unter 2 Minuten beträgt.

6. Verfahren nach einem der Ansprüche 1 bis 5,
**dadurch gekennzeichnet,**
**dass** die Temperatur in der Verfahrensstufe b) 40 bis 180 °C beträgt.

7. Verfahren nach einem der Ansprüche 1 bis 6,
**dadurch gekennzeichnet,**
**dass** der Druck in der Verfahrensstufe b) 0.01 bis 1 bar beträgt.

8. Verfahren nach einem der Ansprüche 1 bis 7,
**dadurch gekennzeichnet,**
**dass** die Sumpffraktion der Verfahrensstufe c) mit einem Kohlenmonoxidpartialdruck von 5 bis 64 bar beaufschlagt wird.

9. Verfahren nach einem der Ansprüche 1 bis 8,
**dadurch gekennzeichnet,**
**dass** als Kohlenmonoxid-haltiges Gas Synthesegas, reines Kohlenmonoxid oder Gemische aus Kohlenmonoxid mit Stickstoff, Methan, Wasserstoff und/oder Kohlendioxid eingesetzt wird.

10. Verfahren nach einem der Ansprüche 1 bis 9,
**dadurch gekennzeichnet,**
**dass** die Sumpffraktion der Verfahrensstufe c) auf Temperaturen von 40 bis 90 °C abgekühlt wird.

11. Verfahren nach einem der Ansprüche 1 bis 10,
**dadurch gekennzeichnet,**
**dass** die Sumpffraktion der Verfahrensstufe c) ganz oder teilweise in den Hydroformylierungsreaktor zurückgeführt wird.

## Claims

1. A process for preparing an aldehyde having from 3 to 21 carbon atoms by hydroformylation of the corresponding olefin using a rhodium catalyst, **characterized in that**
a) the output from the hydroformylation reactor is separated into a gas phase and a liquid phase,
b) the liquid phase is separated into a top fraction containing unreacted olefin and aldehyde and a bottom fraction containing the rhodium catalyst, and
c) the bottom fraction is cooled to below the temperature of the output from the hydroformylation reactor, to a temperature of from 10 to 120°C, and is treated with a gas comprising carbon monoxide with a carbon monoxide partial pressure of from 5 to 300 bar.

2. A process according to claim 1, **characterized in that** a carbon monoxide partial pressure of from 0.5 to 100 bar is set in process stage a).

3. A process according to claim 1 or claim 2, **characterized in that** process stage b) involves a falling film evaporator, a thin film evaporator, a flash vessel or a combination of these units.

4. A process according to any one of claims 1 to 3, **characterized in that** the mean residence time of the liquid phase in process stage b) is less than 15 minutes.

5. A process according to any one of claims 1 to 4, **characterized in that** the mean residence time of the bottom fraction in process stage b) is less than 2 minutes.

6. A process according to any one of claims 1 to 5, **characterized in that** the temperature in process stage b) is from 40 to 180°C.

7. A process according to any one of claims 1 to 6, **characterized in that** the pressure in process stage b) is from 0.01 to 1 bar.

8. A process according to any one of claims 1 to 7, **characterized in that** the bottom fraction is treated with a carbon monoxide partial pressure of from 5 to 64 bar in process stage c).

9. A process according to any one of claims 1 to 8, **characterized in that** synthesis gas, pure carbon monoxide or a mixture of carbon monoxide with nitrogen, methane, hydrogen and/or carbon dioxide is used as gas comprising carbon monoxide.

10. A process according to any one of claims 1 to 9, **characterized in that** the bottom fraction is cooled to temperatures of from 40 to 90°C in process stage c).

11. A process according to any one of claims 1 to 10, **characterized in that** all or some of the bottom fraction from process stage c) is returned to the hydroformylation reactor.

## Revendications

1. Procédé de production d'aldéhydes comprenant de 3 à 21 atomes de carbone par hydroformylation des oléfines correspondantes au moyen de catalyseurs à base de rhodium,
**caractérisé en ce que**
le produit sortant du réacteur d'hydroformylation
a) est séparé en une phase gazeuse et une phase liquide,
b) la phase liquide est séparée en une fraction de tête, contenant les oléfines et les aldéhydes non entrés en réaction, et une fraction de fond contenant le catalyseur à base de rhodium, et
c) la fraction de fond est refroidie en dessous de la température du produit sortant du réacteur d'hydroformylation à une température de 10 à 120°C et soumise, par un gaz contenant du monoxyde de carbone, à une pression partielle du monoxyde de carbone de 5 à 300 bar.

2. Procédé selon la revendication 1,
**caractérisé en ce que**
dans l'étape a) du procédé, une pression partielle du monoxyde de carbone est réglée de 0,5 à 100 bar.

3. Procédé selon l'une quelconque des revendications 1 ou 2,
**caractérisé en ce que**
l'étape b) du procédé présente un évaporateur de film à écoulement vertical, un évaporateur à couche mince, un évaporateur rapide ou une combinaison de ces unités.

4. Procédé selon l'une quelconque des revendications 1 à 3,
**caractérisé en ce que**
le temps de séjour moyen de la phase liquide dans l'étape b) du procédé est inférieur à 15 minutes.

5. Procédé selon l'une quelconque des revendications 1 à 4,
**caractérisé en ce que**
le temps de séjour moyen de la fraction de fond de l'étape b) du procédé est inférieur à 2 minutes.

6. Procédé selon l'une quelconque des revendications 1 à 5,
**caractérisé en ce que**
la température dans l'étape b) du procédé est de 40 à 180°C.

7. Procédé selon l'une quelconque des revendications 1 à 6,
**caractérisé en ce que**
la pression dans l'étape b) du procédé est de 0,01 à 1 bar.

8. Procédé selon l'une quelconque des revendications 1 à 7,
**caractérisé en ce que**
la fraction de fond de l'étape c) du procédé est soumise à une pression partielle du monoxyde de carbone de 5 à 64 bar.

9. Procédé selon l'une quelconque des revendications 1 à 8,
**caractérisé en ce que**
comme gaz contenant du monoxyde de carbone, on utilise du gaz de synthèse, du pur monoxyde de carbone ou des mélanges composés de monoxyde de carbone avec de l'azote, du méthane, de l'hydrogène et/ou du dioxyde de carbone.

10. Procédé selon l'une quelconque des revendications 1 à 9,
**caractérisé en ce que**
la fraction de fond de l'étape c) du procédé est refroidie à des températures de 40 à 90°C.

11. Procédé selon l'une quelconque des revendications 1 à 10,
**caractérisé en ce que**
la fraction de fond de l'étape c) du procédé est ramenée en totalité ou en partie dans le réacteur d'hydroformylation.
